(19) Europäisches Patentamt
European Patent Office
Office européen des brevets

(11) Numéro de publication : **0 419 297 B1**

(12) # FASCICULE DE BREVET EUROPEEN

(45) Date de publication du fascicule du brevet : **08.06.94 Bulletin 94/23**

(51) Int. Cl.$^5$ : **C07D 211/90,** C07D 401/12, // A61K31/44

(21) Numéro de dépôt : **90400096.5**

(22) Date de dépôt : **15.01.90**

(54) **Nouveau procédé de séparation d'isomères optiques des dérivés de la dihydro-1,4 pyridine.**

(30) Priorité : **20.09.89 FR 8912323**

(43) Date de publication de la demande : **27.03.91 Bulletin 91/13**

(45) Mention de la délivrance du brevet : **08.06.94 Bulletin 94/23**

(84) Etats contractants désignés : **AT BE CH DE DK ES FR GB GR IT LI LU NL SE**

(56) Documents cités :
**EP-A- 0 089 167**
**EP-A- 0 259 206**

(56) Documents cités :
**JOURNAL OF MEDICINAL CHEMISTRY, vol. 29, no. 9, septembre 1986, pages 1696-1702, American Chemical Society; J.E. ARROWSMITH et al.: "Long-actingdihydropyridine calcium antagonists. 1. 2-alkoxymethyl derivatives incorporating basic Substituents"**

(73) Titulaire : **ADIR ET COMPAGNIE**
**1 rue Carle Hébert**
**F-92415 Courbevoie Cédex (FR)**

(72) Inventeur : **Peglion, Jean Louis**
**5 Allée des Bégonias**
**F-78110 Le Vesinet (FR)**
Inventeur : **Serkiz, Bernard**
**3 Rue Florian**
**F-77170 Servon Brie Comte Robert (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne un nouveau procédé de préparation d'isomères optiques des dérivés de la dihydro-1,4 pyridine.

Certains dérivés de la dihydro-1,4 pyridine possèdent des propriétés pharmacologiques très intéressantes et trouvent leurs applications en thérapeutique, notamment comme inhibiteurs de la pénétration intracellulaire du calcium. Selon la nature des substituants en position 2, 3, 5, 6, ces composés peuvent présenter un centre chiral en position 4. Il est aussi connu que la configuration absolue autour de l'atome de carbone en 4 peut influencer l'activité pharmacologique des composés. Plusieurs méthodes de séparation des isomères optiques sont déjà connues dans la littérature. Toutefois, soit ces méthodes ne sont pas applicables à tous les dérivés de la dihydro-1,4 pyridine et notamment à ceux contenant des groupements éthéroxydes en position 2 (Chem.Pharm.Bull.,1980,23(9),p.2809-2812 et WO 88/07524) soit elles sont longues et avec des rendements peu intéressants (EP 125803) soit mettent en jeu des intermédiaires de réaction difficiles à manipuler (J.Med.Chem.,1986,29,p.1696-1702).

Des isomères optiques de 1,4-dihydropyridines ont également été préparés comme décrit dans les demandes de brevet : EP-A 259 206 et EP-A 089 167. Toutefois, le procédé de préparation, objet de la présente demande, est totalement original vis à vis de ces documents antérieurs, et il conduit, avec de bons rendements, à des stétéoisomères purs, ce qui n'est pas le cas des procédés de dédoublement classiques antérieurement décrits.

La présente invention a plus particulièrement pour objet un procédé de préparation d'isomères optiques des composés de formule générale I :

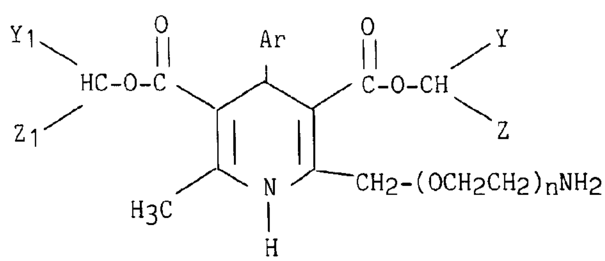

(I)

dans laquelle :

- Ar représente un radical phényle, comportant éventuellement un à cinq substituants identiques ou différents représentant chacun un atome d'halogène, un radical alkoxy renfermant de 1 à 4 atomes de carbone, un radical alkylthio renfermant de 1 à 4 atomes de carbone, un radical trihalogénométhyle, ou un radical méthylènedioxy,
- Y, Z, $Y_1$ et $Z_1$ identiques ou différents représentent chacun un atome d'hydrogène, un radical alkyle inférieur linéaire ou ramifié renfermant de 1 à 4 atomes de carbone, un radical cyclopropyle, un radical dicyclopropylméthyle, un radical dicyclopropyl-2,2 éthyle, un radical dicyclopropyl-2,2 éthylène, un radical dicyclopropyl-3,3 propyle, un radical dicyclopropyl-3,3 propylène-1, un radical alkylène linéaire ou ramifié renfermant de 2 à 5 atomes de carbone ou un radical phényle éventuellement substitué par un radical nitro,

et

- n est égal à 1, 2 ou 3,

caractérisé en ce que l'on condense un composé de formule générale II :

$$OH\text{-}CH_2\text{-}CH_2\text{-}(OCH_2CH_2)_n\text{-}Cl \qquad (II)$$

dans laquelle n a la même signification que pour la formule I,

avec le phtalimide potassique, dans le diméthylformamide, à chaud, pour former un composé de formule III :

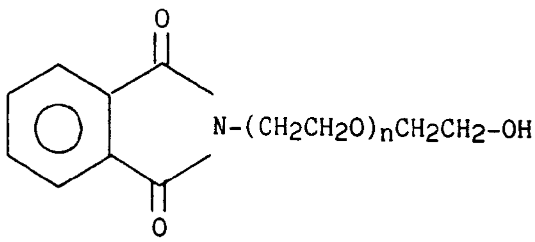

(III)

dans laquelle n a les mêmes valeurs que pour la formule I,
que l'on transforme à l'aide du réactif de Jones en un composé de formule IV :

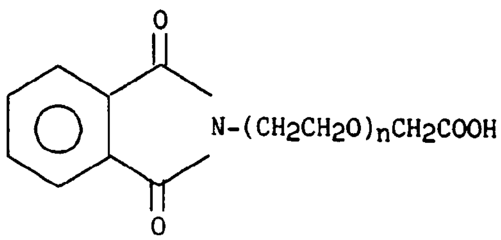

(IV)

dans laquelle n a la même signification que pour la formule I,
que l'on traite avec le carbonyldiimidazole et l'acide de Meldrum en présence de pyridine dans le chlorure de méthylène, pour obtenir le composé de formule V :

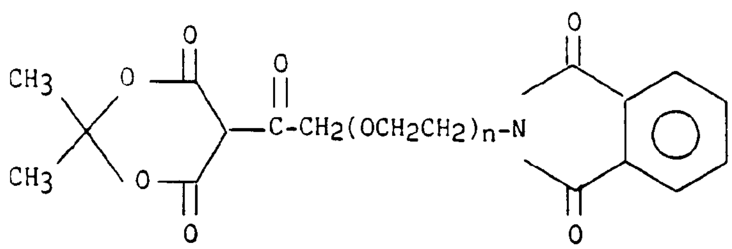

(V)

lequel ensuite on fait réagir avec le (R) phényl-2 méthoxy-2 éthanol, composé de formule VI :

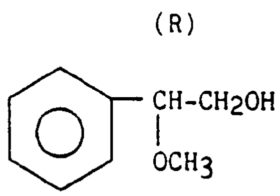

(VI)

pour obtenir un β-cétoester de formule VII :

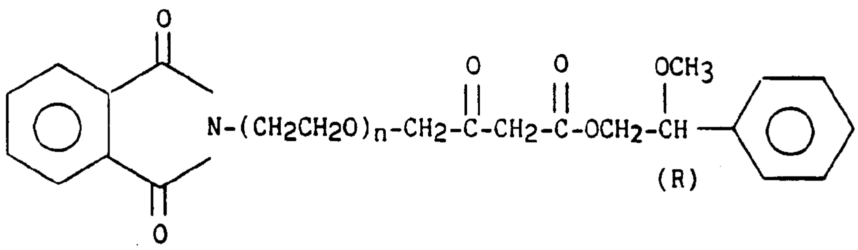

(VII)

dans laquelle n a la même signification que pour la formule I,

qu'on condense en présence de formiate d'ammonium dans l'éthanol, avec un benzylidène de formule VIII :

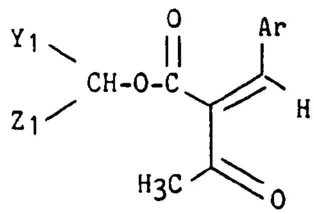

(VIII)

dans laquelle la signification de Ar, $Y_1$, et $Z_1$ reste identique à celle donnée pour la formule I,

pour obtenir un mélange de deux stéréoisomères d'un composé de formule IX :

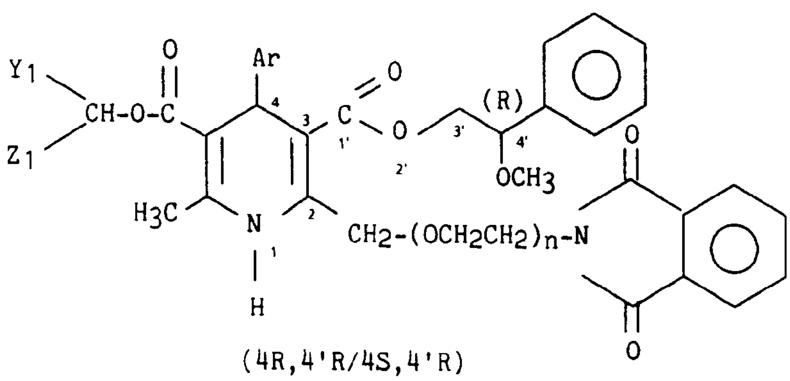

(IX)

(4R,4'R/4S,4'R)

dans laquelle Ar, $Y_1$, $Z_1$ et n ont les mêmes significations que pour la formule I,

lequel ensuite :

soit,

est soumis à l'action d'une solution aqueuse de bicarbonate de sodium, en solution dans l'acétonitrile pour obtenir un mélange de stéréoisomères d'un composé de formule X,

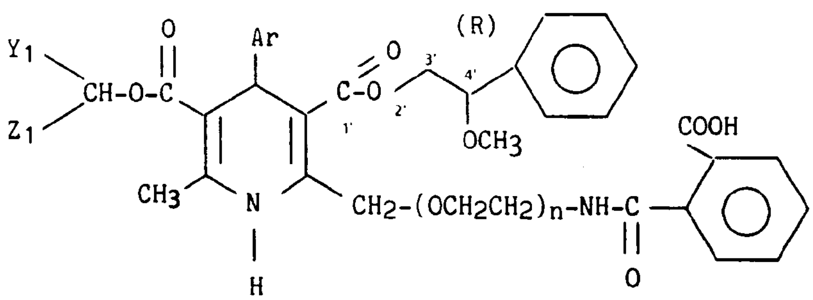

(X)

dans laquelle Ar, $Y_1$, $Z_1$ et n ont les significations indiquées ci-dessus pour la formule I, que l'on sépare

par HPLC en ses deux stéréoisomères, et ensuite que l'on traite chaque stéréoisomère séparément par un mélange de glyme et d'un alcoolate de formule XI :

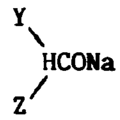

(XI)

dans laquelle la signification de Y et Z est identique à celle donnée pour la formule I,
pour obtenir un mélange contenant l'un de deux stéréoisomères d'un composé de formule XII,

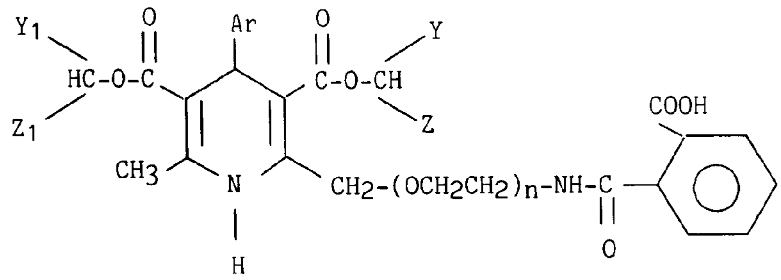

(XII)

dans laquelle Y, Z, $Y_1$, $Z_1$, Ar et n ont les significations données pour la formule I,
ainsi que son homologue substitué en position 5 par un radical éthoxycarbonyle,
soit

le composé de formule IX est séparé en ses deux stéréoisomères par chromatographie sur colonne de silice, et ensuite chaque stéréoisomère est soumis séparément, à l'action d'un alcoolate de formule XI en présence de glyme, en solution dans l'éthanol pour obtenir un mélange contenant un stéréoisomère du composé de formule XII, et son homologue substitué en position 5 par un radical éthoxycarbonyle,

le dit mélange contenant un stéréoisomère de formule (XII) obtenu par l'une des alternatives précédentes, est ensuite soumis à l'action du carbonyldiimidazole en solution dans un alcane halogéné à température ambiante pour obtenir un mélange contenant un stéréoisomère du composé de formule XIII :

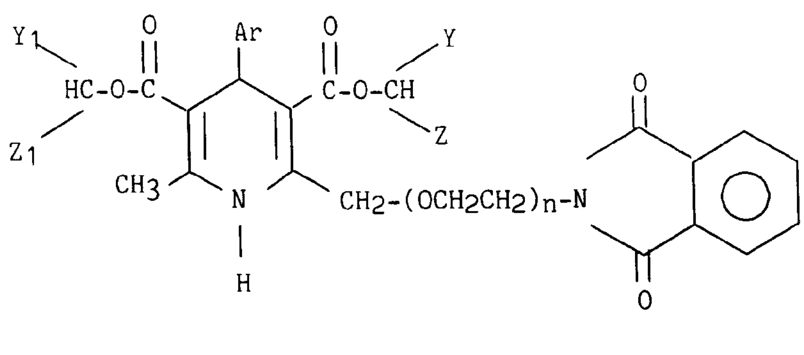

(XIII)

dans laquelle Ar, Y, Z, $Y_1$, $Z_1$, et n ont la même signification que pour la formule I,

et son homologue substitué en 5 par un radical éthoxycarbonyle, lequel mélange est ensuite séparé par HPLC pour obtenir l'un des stéréoisomères du composé de formule XIII pur,

lequel est ensuite porté au reflux dans l'éthanol en présence d'hydrate d'hydrazine pour donner l'un des stéréoisomères du composé de formule I,

qui peut être ensuite salifié avec un acide organique ou minéral pharmaceutiquement compatible pour obtenir les sels d'addition correspondants.

Le (R) phényl-2 méthoxy-2 éthanol composé de formule VI est obtenu par réduction de l'acide correspondant, optiquement actif. Ce dernier composé est préparé selon le procédé décrit dans J.Chem.Soc.,1962,p.1519.

Les composés de la formule VIII peuvent être obtenus par condensation des composés de formule XIV :

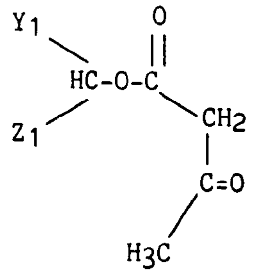

**(XIV)**

dans laquelle la définition de $Y_1$ et $Z_1$ reste identique à celle donnée pour la formule I, avec un aldéhyde aromatique de formule XV :

Ar-CHO **(XV)**

dans laquelle la définition de Ar demeure celle définie précédemment pour la formule I. Cette condensation est réalisée selon la méthode décrite dans Can.J.Chem.,1967,45,p.1001.

Les exemples suivants, donnés à titre non limitatif, illustrent l'invention.

Les points de fusion indiqués sont mesurés selon la technique micro-Köfler.

Les spectres de résonance magnétique nucléaire du proton (R.M.N.) ont été enregistrés à 200 MHz.

La configuration absolue de certains intermédiaires de synthèse n'est pas pour l'instant connue. Dans le cas où les deux isomères d'un intermédiaire ont un pouvoir rotatoire négatif, nous appelons isomère A, le composé qui conduit à l'obtention de l'isomère dextrogyre d'un composé de formule générale I.

## EXEMPLE 1

**Acide [(phtalimido-2 éthoxy)-2 éthoxy]-2 acétique**

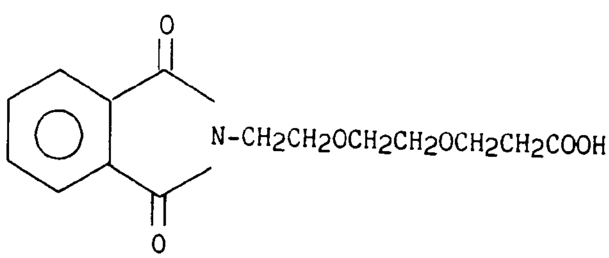

### STADE A

[(phtalimido-2 éthoxy)-2 éthoxy]-2 éthanol

Porter à 95°C, pendant 17 heures, 188 g de [(chloro-2 éthoxy)-2 éthoxy]-2 éthanol, 146 g de phtalimide potassique dans 700 ml de diméthylformamide.

Diluer au chlorure de méthylène, laver par une solution saturée de chlorure de sodium, sécher, évaporer.

Distiller au Kugelrohr $Eb_{0,05}$ mmHg : 180-185°C.

Rendement : 90%

Spectre de résonance magnétique nucléaire du proton (Solvant $CDCl_3$) :

4H(m)7,5 à 8 ppm; 12H(m)3,4 à 4 ppm; 1H (massif échangeable $D_2O$) 2,5 à 3 ppm.

### STADE B

Dissoudre 3 g de l'alcool obtenu au stade précédent dans 150 ml d'acétone. Couler le réactif de Jones en maintenant la température entre 20° et 25°C. Laisser reposer une heure à la température ambiante. Concentrer, diluer ensuite au chlorure de méthylène, laver à l'eau. Sécher et évaporer le solvant pour obtenir le composé attendu.

Point de fusion : 88-90°C

Rendement : 90%

Spectre de résonance magnétique nucléaire du proton (Solvant $CDCl_3$) :

1H (massif échangeable) 8,8 à 9,5 ppm; 4H(m)7,6 à 8,1 ppm; 2H(s)4,1 ppm; 8H(m)3,6 à 4 ppm.

**EXEMPLE 2**

**(R) phényl-2 méthoxy-2 éthanol**

Réduire 72 g d'acide (R) phényl-2 méthoxy-2 acétique (préparé selon la méthode décrite dans J.Chem.Soc., 1962,p.1519), par 16,5 g d'hydrure de lithium et d'aluminium dans 300 ml de tétrahydrofuranne.

Hydrolyser et filtrer les sels minéraux et ensuite distiller l'huile résiduelle au Kugelrohr $Eb_{15\ mmHg}$ = 105°C.

Rendement : 76%

Spectre de résonance magnétique nucléaire du proton (solvant $CDCl_3$) :

5H(m)7,35 ppm; 1H(d)4,35 ppm; 2H(m)3,65 ppm; 3H(s)3,3 ppm; 1H (d échangeable)2,35 ppm.

Pouvoir rotatoire en solution à 1% dans l'éthanol :

| λ(nm) | [α]24°C |
|---|---|
| 589 | - 122° |
| 578 | - 127° |
| 546 | - 145° |
| 436 | - 249° |
| 365 | - 396° |

**EXEMPLE 3**

**Ester (R) phényl-2 methoxy-2 éthylique de l'acide dioxa-5,8 oxo-3 phtalimido-10 décanoïque**

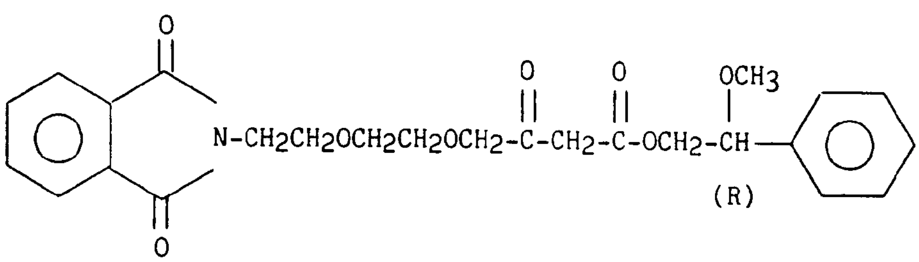

A une suspension contenant 34,7 g du composé de l'exemple 1 dans 210 ml de chlorure de méthylène, on ajoute en une fois 20 g de carbonyldiimidazole.

Agiter jusqu'à la fin de dégagement gazeux. Couler ensuite en un goutte à goutte rapide un mélange constitué de 17,7 g d'acide de Meldrum et 9,2 g de pyridine dans 70 ml de chlorure de méthylène. Agiter sous azote pendant une nuit.

Transvaser dans une ampoule à décanter, laver à l'acide sulfurique N jusqu'à pH acide, puis une fois à l'eau, sécher et évaporer le solvant.

Chauffer l'huile obtenue au bain marie avec 25 g de l'alcool obtenu dans l'exemple 2, jusqu'à fin de dégagement.

Soumettre le milieu réactionnel à une chromatographie sur colonne (chromatographie flash) contenant 1,8 kg de silice en utilisant comme éluant un mélange de cyclohexane et d'acétate d'éthyle (1:1 V/V) pour obtenir le composé attendu.

Rendement : 70%

Spectre de résonance magnétique nucléaire du proton (Solvant $CDCl_3$) :

4H(m)7,6 à 8,1 ppm; 5H(s)7,35 ppm; 3H(m)4 à 4,6 ppm; 2H(s)4,1 ppm; 10H(m)3,5 à 4 ppm; 3H(s)3,3 ppm.

Pouvoir rotatoire en solution à 1% dans l'éthanol :

| λ(nm) | [α]21°C |
|---|---|
| 589 | - 35,1° |
| 578 | - 36,5° |
| 546 | - 41,4° |
| 436 | - 69,8° |
| 365 | - 107,2° |

**EXEMPLE 4**

**Ester méthylique de l'acide (dichloro-2,3 benzylidène)-2 oxo-3 butanoïque**

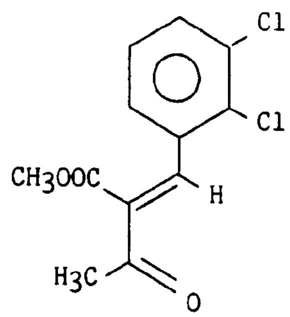

Porter au reflux sous agitation pendant 4 heures, un mélange contenant 8,7 g de dichloro-2,3 benzaldéhyde, 5,8 g d'acétoacétate de méthyle, 28 gouttes de pyridine et 38 gouttes d'acide hexanoïque dans 280 ml de benzène. Transvaser dans une ampoule, laver avec une solution à 10% de bicarbonate de sodium, puis avec une solution d'acide chlorhydrique N, puis à l'eau. Sécher et évaporer. Les cristaux obtenus sont lavés par l'éther isopropylique.

Rendement : 65%

Spectre de résonance magnétique nucléaire du proton (Solvant $CDCl_3$) :

1H(2s)8 et 8,05 ppm; 3H(m)7,1 à 7,8 ppm; 3H(2s)3,9 et 3,75 ppm; 3H(2s)2,45 et 2,2 ppm.

**EXEMPLE 5**

**(4R,4'R/4S,4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine**

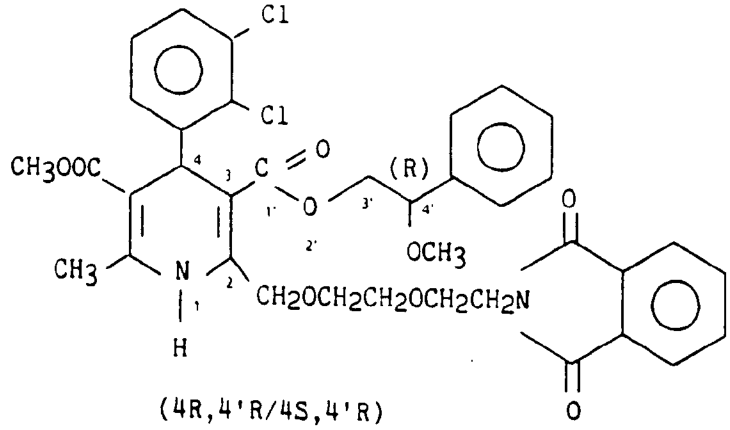

(4R,4'R/4S,4'R)

Agiter sous azote à 40°C pendant 48 heures un mélange contenant 22,2 g du composé décrit dans l'exemple 4, 38 g du composé décrit dans l'exemple 3, et 6,3 g de formiate d'ammonium dans 200 ml d'éthanol. Le milieu résiduel est évaporé et purifié sur une colonne contenant 4 kg de silice en utilisant comme éluant un mélange constitué de chlorure de méthylène et d'acétate d'éthyle (9:1 V/V/).

Spectre de résonance magnétique nucléaire du proton (Solvant $CDCl_3$) :

2H(m)7,8 ppm; 2H(m)7,7 ppm; 7H(m)7,3 à 7,1 ppm; 1H(t)7,05 ppm; 1H(s)5,45 ppm; 2H(m)4,6 ppm; 11H(m)3,6 à 4,4 ppm; 3H(2s)3,6 ppm; 3H(2s)3,2 et 3,05 ppm; 3H(s)2,3 ppm; 1H(s) non échangeable par $D_2O$ 7,4 ppm.

Pouvoir rotatoire en solution à 1% dans le chloroforme :

| λ(nm) | [α]21°C |
|---|---|
| 589 | - 13,3° |
| 578 | - 13,8° |
| 546 | - 15,1° |

**EXEMPLE 6**

**(4R4'R/4S4'R) {[[(carboxy-2 phénylcarboxamido)-2 éthoxy]-2 éthoxy] méthyl}-2 (dichloro-2,3 phényl)-4 méthoxycarbonyl-5 (méthoxy-2 phényl-2 éthoxycarbonyl)-3 méthyl-6 dihydro-1,4 pyridine**

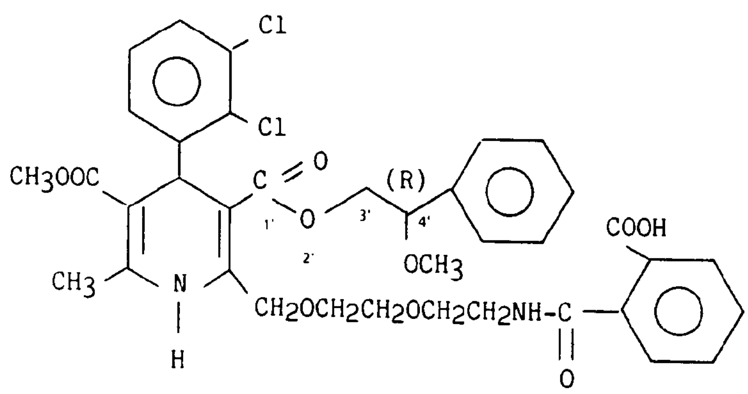

(4R,4'R/4S,4'R)

Porter au reflux sous agitation pendant 24 heures un mélange contenant 16,5 g du composé de l'exemple 5, 100 ml de solution aqueuse à 10% de bicarbonate de sodium et 230 ml d'acétonitrile. Evaporer le solvant, reprendre à l'eau, acidifier à l'acide chlorhydrique N et extraire pour obtenir le composé attendu.

Rendement : 87%

Spectre de résonance magnétique nucléaire du proton (Solvant $CDCl_3$) :

1H(m)7,9 ppm; 3H(m)7,5 ppm; 7H(m)7,4 à 7,15 ppm; 1H(t)7,05 ppm; 1H(2s)5,45 ppm; 2H(m)4,8 ppm; 2H(m)4,4 à 4,1 ppm; 1H(m)3,9 ppm; 8H(m)3,8 à 3,5 ppm; 3H(2s)3,6 ppm; 3H(2s)3,2 et 3,05 ppm; 3H(s)2,3 ppm; 1H(t)6,55 ppm; 1H (signal masqué échangé par $D_2O$) 7,45 ppm; 1H (signal plat) 7 à 5 ppm échangé par $D_2O$.

Pouvoir rotatoire en solution à 1% dans le chloroforme :

| λ(nm) | [α]21°C |
|---|---|
| 589 | - 14,2° |
| 578 | - 14,8° |
| 546 | - 16,3° |

**EXEMPLE 7**

**Séparation des deux isomères de la (4R4'R/4S4'R) {[[(carboxy-2 phénylcarboxamido)-2 éthoxy]-2 éthoxy] méthyl}-2 (dichloro-2,3 phényl)-4 méthoxycarbonyl-5 (méthoxy-2 phényl-2 éthoxycarbonyl)-3 méthyl-6 dihydro-1,4 pyridine**

Le composé de l'exemple 6 est mis en solution dans l'acétonitrile et séparé en ses deux isomères par HPLC préparative en utilisant une colonne Lichroprep RP18 et comme éluant un mélange de méthanol et de phosphate disodique 0,025M (55:45 V/V), débit 3 ml/min. Détection à 350 nm.

Isomère A

L'isomère A est obtenu après environ 20 minutes d'élution.
Pouvoir rotatoire en solution à 1% dans le chloroforme :

| λ(nm) | [α]21°C |
|---|---|
| 589 | - 19,3° |
| 578 | - 20,0° |
| 546 | - 21,9° |

Isomère B

L'isomère B est obtenu selon le procédé décrit ci-dessus après environ 23 minutes d'élution.

**EXEMPLE 8**

**Séparation des deux isomères de la (4R4'R/4S4'R) (dichloro-2,3 phényl)-4 méthoxycarbonyl-5 (méthoxy-2 phényl-2 éthoxycarbonyl)-3 méthyl-6 {[(phtalimido-2 éthoxy)-2 éthoxy] méthyl}-2 dihydro-1,4 pyridine**

Isomère B

Le composé de l'exemple 5 est chromatographié sur une colonne préparative contenant 4 kg de silice, en utilisant comme éluant un mélange de chlorure de méthylène et d'acétate d'éthyle (95:5 V/V). Isoler le premier composé issu de la séparation qui est le moins polaire.
Rendement : 20%
Spectre de résonance magnétique nucléaire du proton (Solvant $CDCl_3$) :
2H(m)7,8 ppm; 2H(m)7,7 ppm; 7H(m)7,4 à 7,1 ppm; 1H(t)7,05 ppm; 1H(s),5,45 ppm; 2H(m)4,4 ppm; 11H(m)4 à 3,7 ppm; 3H(s)3,6 ppm; 3H(s)3,05 ppm; 3H(s)2,3 ppm; 1H(s)7,45 ppm (difficilement échangeable par $D_2O$).
Pouvoir rotatoire en solution à 1% dans le chloroforme :

| λ(nm) | [α]21°C |
|---|---|
| 589 | - 9,0° |
| 578 | - 9,6° |
| 546 | - 10,4° |

Isomère A

Le deuxième composé, le plus polaire, issu de la séparation indiquée ci-dessus est l'isomère A. Pouvoir rotatoire en solution à 1% dans le chloroforme :

| λ(nm) | [α]21°C |
|---|---|
| 589 | - 19,1° |
| 578 | - 19,9° |
| 546 | - 22,0° |

EXEMPLE 9

**Mélange de (-) {[[(carboxy-2 phényl carboxamido)-2 éthoxy]-2 éthoxy] méthyl}-2 (dichloro-2,3 phényl)-4 éthoxycarbonyl-3 méthoxycarbonyl-5 méthyl-6 dihydro-1,4 pyridine et de son homologue substitué en position 5 par un radical éthoxycarbonyle**

* Premier procédé
  3 g de l'isomère B de l'exemple 7 sont portés au reflux avec 30 ml de glyme et 42,9 ml d'une solution éthanolique d'éthanolate de sodium 0,26M. Evaporer, reprendre à l'eau, acidifier et extraire à l'acétate d'éthyle pour obtenir les composés attendus.
* Deuxième procédé
  Porter à reflux 3 g de l'isomère B, obtenus à l'exemple 8, avec 30 ml de glyme et 28,6 ml d'une solution éthanolique d'éthanolate de sodium 0,26M. Evaporer, reprendre à l'eau, acidifier et extraire à l'acétate d'éthyle pour obtenir les composés attendus.
  Rendement 65%

EXEMPLE 10

**Mélange de (+) {[[(carboxy-2 phényl carboxamido)-2 éthoxy]-2 éthoxy] méthyl}-2 (dichloro-2,3 phényl)-4 éthoxycarbonyl-3 méthoxycarbonyl-5 méthyl-6 dihydro-1,4 pyridine et de son homologue substitué en position 5 par un radical éthoxycarbonyle**

Traiter l'isomère A de l'exemple 7 selon le premier procédé décrit dans l'exemple 9 ou l'isomère A obtenu dans l'exemple 8 selon le deuxième procédé décrit dans l'exemple 9 pour obtenir les composés attendus.

EXEMPLE 11

**Mélange de (-) (dichloro-2,3 phényl)-4 éthoxycarbonyl-3 méthoxycarbonyl-5 méthyl-6 {[phtalimido-2 éthoxy)-2 éthoxy] méthyl}-2 dihydro-1,4 pyridine et de son homologue substitué en 5 par un radical éthoxycarbonyle**

Dissoudre 1,9 g du mélange obtenu dans l'exemple 9 dans 30 ml de chlorure de méthylène et ajouter en une fois 0,9 g de carbonyldiimidazole; Laisser sous agitation pendant une nuit.

Transvaser le milieu réactionnel dans une ampoule à décanter, laver au bicarbonate de sodium à 10% et ensuite à l'acide chlorhydrique N et à l'eau. Sécher et évaporer pour obtenir les composés attendus.
Rendement : 65%

**EXEMPLE 12**

**Mélange de (+) (dichloro-2,3 phényl)-4 éthoxycarbonyl-3 méthoxycarbonyl-5 méthyl-6 {[phtalimido-2 éthoxy)-2 éthoxy] méthyl}-2 dihydro-1,4 pyridine et de son homologue substitué en 5 par un radical éthoxycarbonyle**

Dissoudre 0,65 g du mélange obtenu dans l'exemple 10 dans 10 ml de chlorure de méthylène et ajouter en une fois 0,3 g de carbonyldiimidazole. Procéder comme il est indiqué dans l'exemple 11 pour obtenir les composés attendus.

**EXEMPLE 13**

**(-) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine**

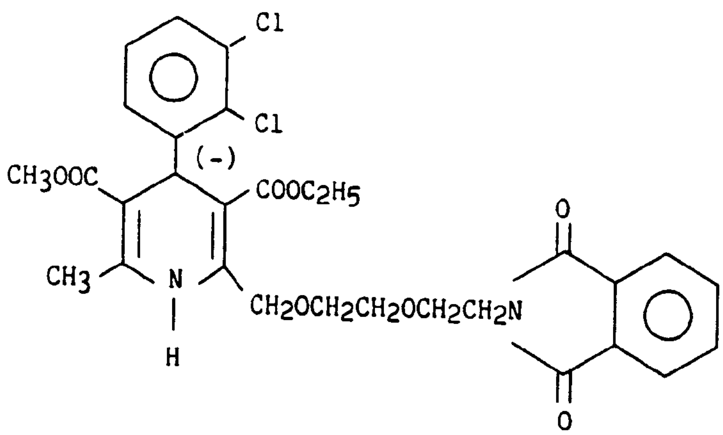

Séparer le mélange obtenu dans l'exemple 11 par HPLC préparative en utilisant une colonne Lichroprep RP 18 de 50 cm de long et comme éluant un mélange d'éthanol, d'eau et de TFA (500:500:1). Le composé attendu est isolé en premier.
Rendement : 30%
Pouvoir rotatoire en solution à 1% dans le DMSO :

| λ(nm) | [α]20°C |
|---|---|
| 589 | - 34,6° |
| 578 | - 36,5° |
| 546 | - 43,9° |
| 436 | - 119,0° |

**EXEMPLE 14**

**(+) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine**

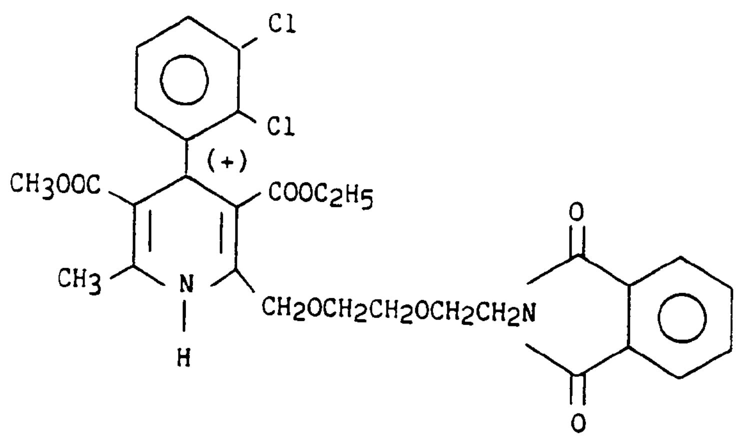

Ce composé a été obtenu selon le procédé décrit dans l'exemple 13, à partir du mélange de l'exemple 12. Pouvoir rotatoire en solution à 1% dans le DMSO:

| λ(nm) | [α]20°C |
|---|---|
| 589 | + 27,8° |
| 578 | + 30,2° |
| 546 | + 37,4° |
| 436 | + 123,5° |

**EXEMPLE 15**

**(-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

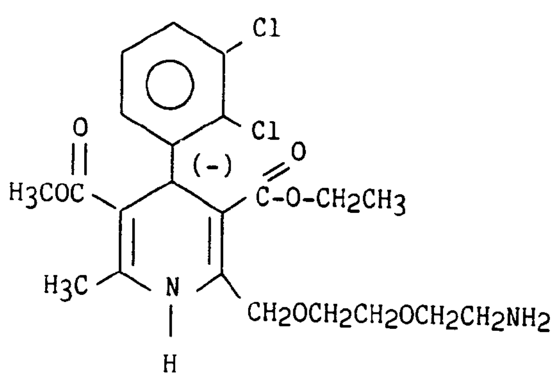

Mettre à reflux 1 g du composé préparé dans l'exemple 13 avec 10 ml d'éthanol et 0,25 ml d'hydrate d'hydrazine pendant 4 heures. Evaporer le solvant, reprendre à l'éther éthylique, laver avec 5 ml de soude normale et épuiser la phase éthérée à l'acide chlorhydrique N. Les phases aqueuses sont ensuite basifiées et extraites

à l'éther éthylique pour obtenir le composé attendu.
Rendement : 60%
Point de fusion : 69-71°C
Spectre de résonance magnétique nucléaire du proton (Solvant $CDCl_3$) :
1H(m)7,3 à 7,7 ppm échangeable par $D_2O$; 3H(m)7,6 à 6,9 ppm; 1H(s)5,5 ppm; 2H(s)4,8 ppm; 2H(q)4 ppm; 4H(s)3,7 ppm; 2H(m)3,4 à 3,7 ppm; 3H(s)3,6 ppm; 2H(t)2,9 ppm; 3H(s)2,3 ppm; 2H(m)échangeables par $D_2O$ 1,4 à 1,8 ppm; 3H(t)1,2 ppm.
Pouvoir rotatoire en solution à 1% dans le chloroforme :

| λ(nm) | [α]20,5°C |
|---|---|
| 589 | - 36,5° |
| 578 | - 38,7° |
| 546 | - 46,8° |
| 436 | - 133,0° |

**EXEMPLE 16**

**(+) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

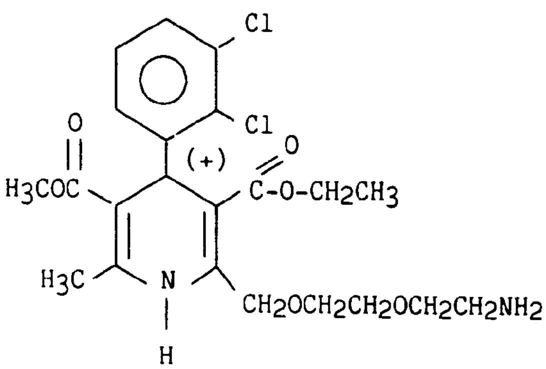

Ce composé a été préparé à partir de la (+) (dichloro-2,3 phényl)-4 éthoxycarbonyl-3 méthoxycarbonyl-5 méthyl-6 {[(phtalimido-2 éthoxy)-2 éthoxy] méthyl}-2 dihydro-1,4 pyridine avec le procédé décrit dans l'exemple 15.
Point de fusion : 68-70°C
Spectre de résonance magnétique nucléaire du proton (Solvant $CDCl_3$) :
2H(m)7,3 ppm; 1H(t)7,1 ppm; 1H(s)5,5 ppm; 2H(m,syst A B)4,8 ppm; 2H(q)4,05 ppm; 4H(m)3,7 ppm; 3H(s)3,65 ppm; 2H(t)3,6 ppm; 2H(t)2,9 ppm; 3H(s)2,3 ppm; 3H(t)1,2 ppm; [1H(s)7,4 ppm; 2H(s)1,65 ppm échangés par $D_2O$]
Pouvoir rotatoire en solution à 1% dans le chloroforme :

| λ(nm) | [α]20°C |
|---|---|
| 589 | - 36,3° |
| 578 | - 38,9° |
| 546 | - 46,9° |
| 436 | - 134,0° |

**EXEMPLE 17**

**Fumarate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

Le fumarate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine est obtenu après solubilisation de 4,2 g du composé de l'exemple 15 dans 50 ml d'une solution éthanolique d'acide fumarique 0,172M. Recristallisation dans l'acétonitrile.
Rendement : 92%
Point de fusion : 115°C
Spectre de résonance magnétique nucléaire du proton (Solvant $CDCl_3$et DMSO-$d_6$) : 2H(2dd)7,3 ppm; 1H(t)7,1 ppm; 2H(s)6,7 ppm; 1H(s)5,45 ppm; 2H(m)4,7 ppm; 2H(q)4 ppm; 6H(m)5,7 ppm; 3H(s)3,6 ppm; 2H(m)3,1 ppm; 3H(s)2,3 ppm; 3H(t)1,3 ppm; 1H(s échangé $D_2O$)7,7 ppm; 4H(s échangé $D_2O$)5,7 ppm.
Pouvoir rotatoire en solution à 1% dans le DMSO :

| λ(nm) | [α]20,5°C |
|---|---|
| 589 | - 33,1° |
| 578 | - 35,2° |
| 546 | - 43,0° |
| 436 | - 134,6° |

**EXEMPLE 18**

**Fumarate de la (+) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

5,45 g de base décrite dans l'exemple 16, 1,3 g d'acide fumarique sont dissous à chaud dans 100 ml d'éthanol.

Après évaporation, on concrétise par 3 ml d'acétate d'éthyle, sèche et recristallise de 80 cc d'acétonitrile.

On obtient 6,2 g du composé attendu.

Point de fusion : 115°C
Spectre de résonance magnétique nucléaire du proton (Solvant $CDCl_3$) : 2H(2dd)7,3 ppm; 1H(t)7,1 ppm; 2H(s)6,7 ppm; 1H(s)5,45 ppm; 2H(m syst A B)4,7 ppm; 2H(q)4 ppm; 6H(m)3,7 ppm; 3H(s)3,6 ppm; 2H(m)3,1 ppm; 3H(s)2,3 ppm; 3H(t)1,3 ppm; 1H(s)7,7 ppm; 4H(s)5,7 ppm.
Pouvoir rotatoire en solution à 1% dans l'éthanol :

| λ(nm) | [α]20,5°C |
|---|---|
| 589 | + 32,9° |
| 578 | + 35,2° |
| 546 | + 43,2° |
| 436 | + 135,4° |

**EXEMPLE 19**

**(+) Tartrate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

0,2 g du composé de l'exemple 15 sont dissous dans 3,1 ml de solution éthanolique d'acide (+) tartrique 0,133M. Après évaporation du solvant, on obtient 0,24 g du sel attendu.
Point de fusion : 150°C
Pouvoir rotatoire en solution à 1% dans le DMSO:

| λ(nm) | [α]21,5°C |
|---|---|
| 589 | - 29,9° |
| 578 | - 32,5° |
| 546 | - 40,5° |
| 436 | - 133,9° |

**EXEMPLE 20**

**(-) Tartrate de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

0,3 g du composé de l'exemple 15 sont dissous dans 4,6 ml d'une solution éthanolique d'acide (-) tartrique 0,133M. On isole le sel attendu après filtration.
Point de fusion : 161-166°C (sublimation)
Pouvoir rotatoire en solution à 1% dans le DMSO:

| λ(nm) | [α]21,5°C |
|---|---|
| 589 | - 32,9° |
| 578 | - 35,0° |
| 546 | - 42,8° |
| 436 | - 142,4° |

**EXEMPLE 21**

**Tartrate racémique de la (-) {[(amino-2 éthoxy)-2 éthoxy] méthyl}-2 (dichloro-2,3 phényl)-4 éthoxycarbonyl-3 méthoxycarbonyl-5 méthyl-6 dihydro-1,4 pyridine**

0,45 g du composé de l'exemple 15 sont dissous dans 6,9 ml d'une solution éthanolique d'acide tartrique racémique 0,133M.

On isole le sel attendu après filtration.

Point de fusion : 160-170°C

Pouvoir rotatoire en solution à 1% dans le DMSO:

| λ(nm) | [α]21,5°C |
|---|---|
| 589 | - 31,2° |
| 578 | - 33,5° |
| 546 | - 41,1° |
| 436 | - 135,9° |

**Revendications**

1. Procédé de préparation d'isomères optiques des composés de formule générale I :

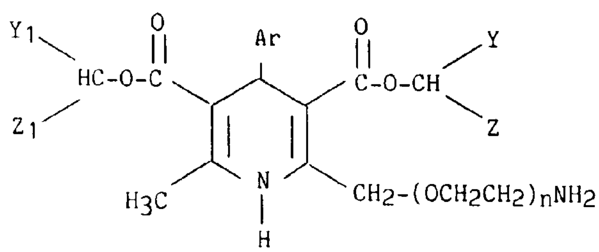

(I)

dans laquelle :

- Ar représente un radical phényle, comportant éventuellement un à cinq substituants identiques ou différents représentant chacun un atome d'halogène, un radical alkoxy renfermant de 1 à 4 atomes de carbone, un radical alkylthio renfermant de 1 à 4 atomes de carbone, un radical trihalogénométhyle, ou un radical méthylènedioxy,
- Y, Z, $Y_1$ et $Z_1$ identiques ou différents représentent chacun un atome d'hydrogène, un radical alkyle inférieur linéaire ou ramifié renfermant de 1 à 4 atomes de carbone, un radical cyclopropyle, un radical dicyclopropylméthyle, un radical dicyclopropyl-2,2 éthyle, un radical dicyclopropyl-2,2 éthylène, un radical dicyclopropyl-3,3 propyle, un radical dicyclopropyl-3,3 propylène-1, un radical alkylène linéaire ou ramifié renfermant de 2 à 5 atomes de carbone ou un radical phényle éventuellement substitué par un radical nitro,

et

- n est égal à 1, 2 ou 3,

caractérisé en ce que l'on condense un composé de formule générale II :

$$OH\text{-}CH_2\text{-}CH_2\text{-}(OCH_2CH_2)_n\text{-}Cl \qquad (II)$$

dans laquelle n a la même signification que pour la formule I,

avec le phtalimide potassique, dans le diméthylformamide à chaud, pour former un composé de formule III :

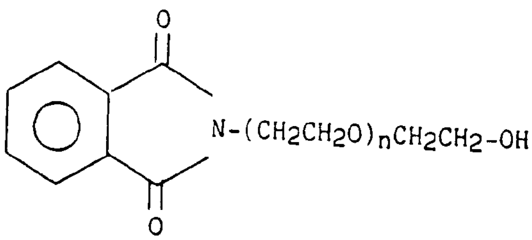

(III)

dans laquelle n a les mêmes valeurs que pour la formule I,

que l'on transforme à l'aide du réactif de Jones en un composé de formule IV :

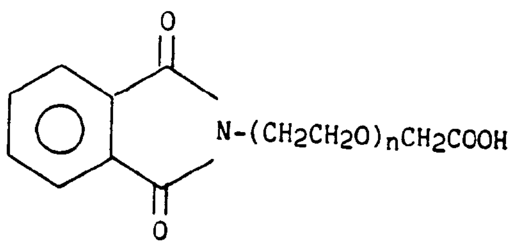

(IV)

dans laquelle n a la même signification que pour la formule I,

que l'on traite avec le carbonyldiimidazole et l'acide de Meldrum en présence de pyridine dans le chlorure de méthylène, pour obtenir le composé de formule V :

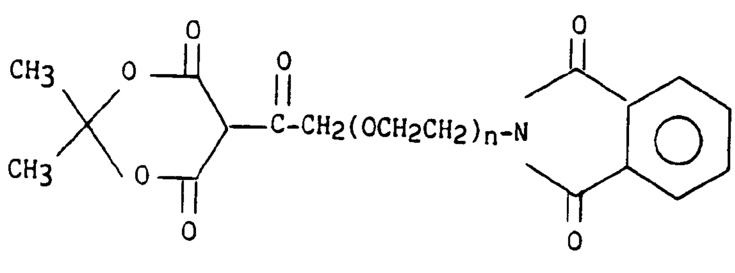

(V)

lequel ensuite on fait réagir avec le (R) phényl-2 méthoxy-2 éthanol, composé de formule VI :

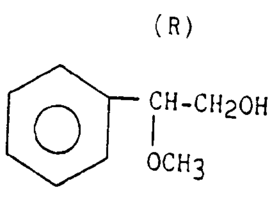

(VI)

pour obtenir un β-cétoester de formule VII :

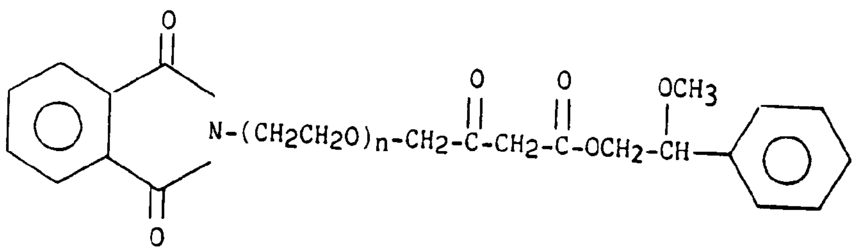

(VII)

dans laquelle n a la même signification que pour la formule I,

qu'on condense en présence de formiate d'ammonium dans l'éthanol, avec un benzylidène de formule VIII :

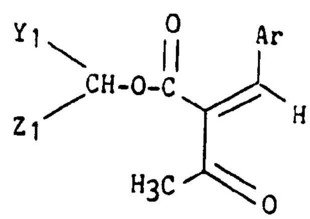

(VIII)

dans laquelle la signification de Ar, $Y_1$, et $Z_1$ reste identique à celle donnée pour la formule I, pour obtenir un mélange de deux stéréoisomères d'un composé de formule IX :

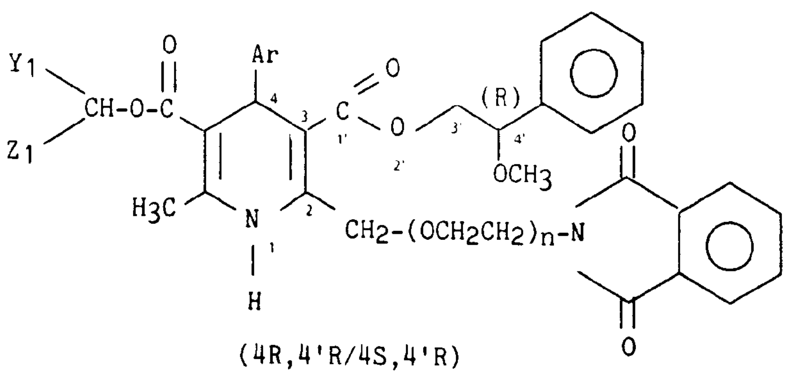

(IX)

(4R,4'R/4S,4'R)

dans laquelle Ar, $Y_1$, $Z_1$ et n ont les mêmes significations que pour la formule I,

lequel ensuite :

soit,

est soumis à l'action d'une solution aqueuse de bicarbonate de sodium, en solution dans l'acétonitrile pour obtenir un mélange de stéréoisomères d'un composé de formule X,

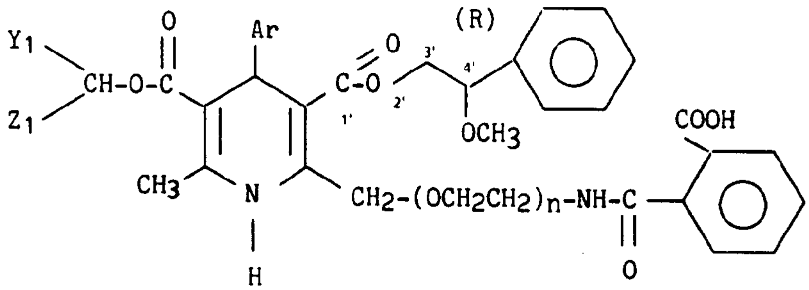

(X)

(4R,4'R/4S,4'R)

dans laquelle Ar, $Y_1$, $Z_1$ et n ont les significations indiquées ci-dessus pour la formule I, que l'on sépare par HPLC en ses deux stéréoisomères, et ensuite que l'on traite chaque stéréoisomère séparément par un mélange de glyme et d'un alcoolate de formule XI :

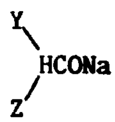

(XI)

dans laquelle la signification de Y et Z est identique à celle donnée pour la formule I,
pour obtenir un mélange contenant l'un de deux stéréoisomères d'un composé de formule XII,

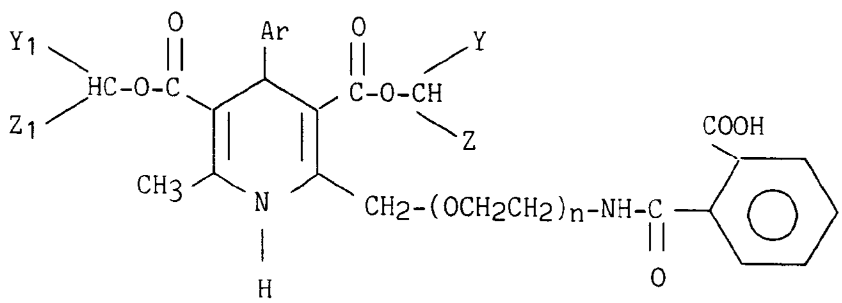

(XII)

dans laquelle Y, Z, $Y_1$, $Z_1$, Ar et n ont les significations données pour la formule I,
ainsi que son homologue substitué en position 5 par un radical éthoxycarbonyle,
<u>soit</u>
le composé de formule IX est séparé en ses deux stéréoisomères par chromatographie sur colonne de silice, et ensuite chaque stéréoisomère est soumis séparément, à l'action d'un alcoolate de formule XI en présence de glyme, en solution dans l'éthanol pour obtenir un mélange contenant un stéréoisomère du composé de formule XII, et son homologue substitué en position 5 par un radical éthoxycarbonyle,

le dit mélange, contenant un stétéoisomère de formule (XII) obtenu par l'une des alternatives précédentes, est ensuite soumis à l'action du carbonyldiimidazole en solution dans un alcane halogéné à température ambiante pour obtenir un mélange contenant un stéréoisomère du composé de formule XIII :

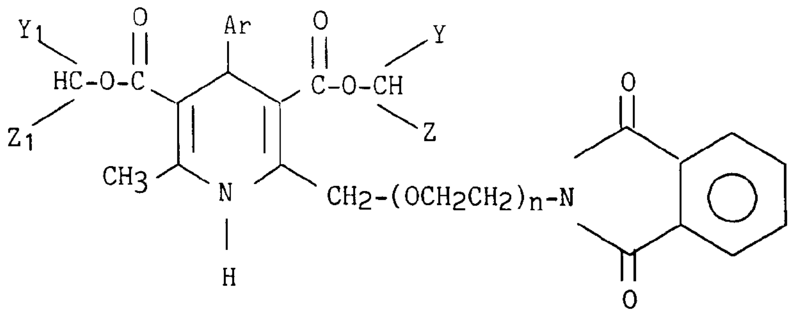

(XIII)

dans laquelle Ar, Y, Z, $Y_1$, $Z_1$, et n ont la même signification que pour la formule I,

et son homologue substitué en 5 par un radical éthoxycarbonyle, lequel mélange est ensuite séparé par HPLC pour obtenir l'un des stéréoisomères du composé de formule XIII pur,

lequel est ensuite porté au reflux dans l'éthanol en présence d'hydrate d'hydrazine pour donner l'un des stéréoisomères du composé de formule I,

qui peut être ensuite salifié avec un acide organique ou minéral pharmaceutiquement compatible pour obtenir les sels d'addition correspondants.

**2.** Les stéréoisomères purs d'un composé de formule X :

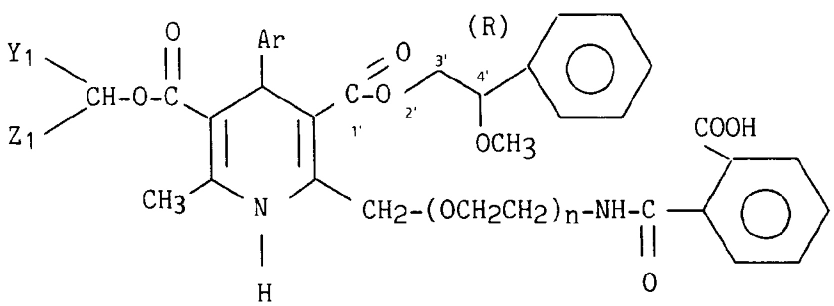

(X)

(4R,4'R/4S,4'R)

dans laquelle Ar, $Y_1$, $Z_1$ et n ont la même signification que pour la formule I, selon la revendication 1, intermédiaires utiles du procédé de préparation selon la revendication 1.

**3.** Procédé selon la revendication 1 caractérisé en ce que l'on obtient un composé de formule XIII:

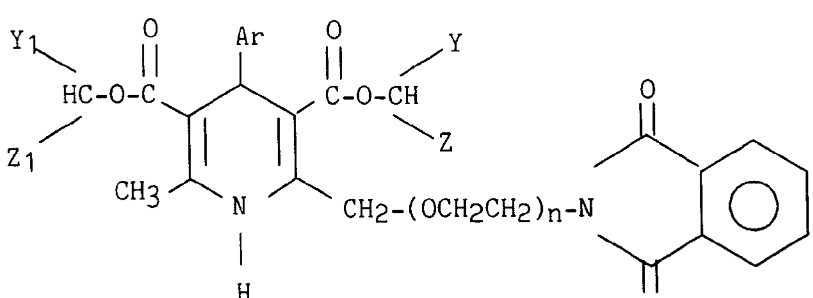

(XIII)

dans laquelle Ar, Y, Z, $Y_1$, $Z_1$ et n ont la même signification que pour la formule I selon la revendication 1,

en soumettant un composé de formule XII :

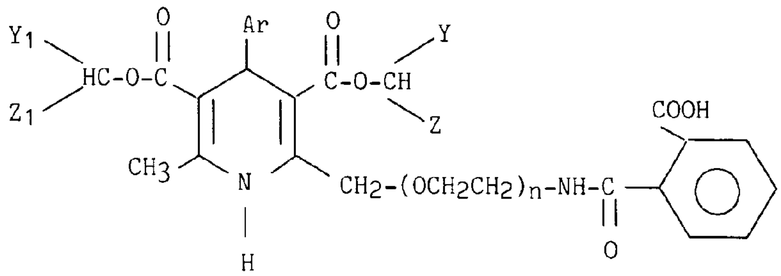

(XII)

dans laquelle Y, Z, $Y_1$, $Z_1$, Ar et n ont les significations données pour la formule I selon la revendication 1,

à l'action du carbonyldiimidazole en solution dans un alcane halogéné tel que le chlorure de méthylène à température ambiante.

**Patentansprüche**

1. Verfahren zur Herstellung von optischen Isomeren von Verbindungen der allgemeinen Formel I :

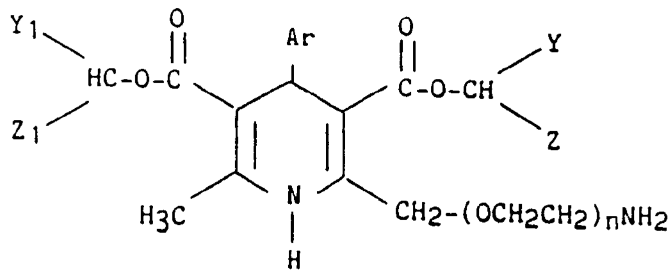

(I)

in der

- Ar eine Phenylgruppe, die gegebenenfalls einen bis fünf Substituenten, die gleichartig oder verschieden sein können, und jeweils ein Halogen atom, eine Alkoxygruppe mit 1 bis 4 Kohlenstoffatomen, eine Alkylthiogruppe mit 1 bis 4 Kohlenstoffatomen, eine Trihalogenomethylgruppe oder eine Methylendioxygruppe darstellen, aufweist,
- Y, Z, $Y_1$ und $Z_1$, die gleichartig oder verschieden sein können, jeweils ein Wasserstoffatom, eine niedrigmolekulare, geradkettige oder verzweigte Alkylgruppe mit 1 bis 4 Kohlenstoffatomen, eine Cyclopropylgruppe, eine Dicyclopropylmethylgruppe, eine 2,2-Dicyclopropylethylgruppe, eine 2,2-Dicyclopropyl-ethylengruppe, eine 3,3-Dicyclopropyl-propylgruppe, eine 3,3-Dicyclopropyl-propylen-1-gruppe, eine geradkettige oder verzweigte Alkylengruppe mit 2 bis 5 Kohlenstoffatomen oder eine Phenylgruppe, die gegebenenfalls durch eine Nitrogruppe substituiert ist,

und

- n 1, 2 oder 3 bedeuten,

**dadurch gekennzeichnet,** daß man eine Verbindung der allgemeinen Formel II:

$$OH-CH_2-CH_2-(OCH_2CH_2)_n-Cl \qquad (II)$$

in der n die bezüglich der Formel I angegebenen Bedeutungen besitzt,

mit Kalium-phthalimid in warmem Dimethylformamid kondensiert zur Bildung einer Verbindung der Formel III:

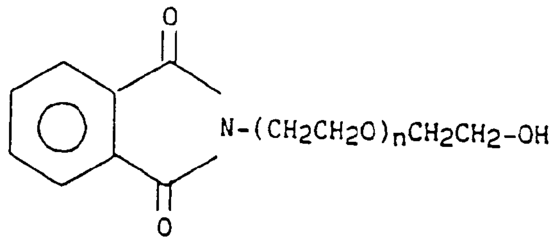

(III)

in der n die bezüglich der Formel I angegebenen Bedeutungen besitzt, welche man mit Hilfe des Jones-Reagens in eine Verbindung der Formel IV:

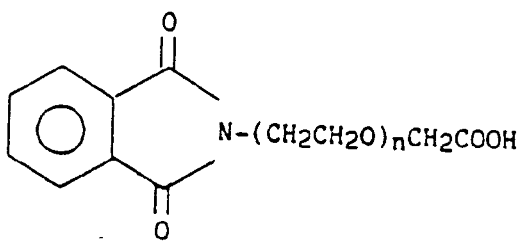

(IV)

umwandelt, in der n die bezüglich der Formel I angegebenen Bedeutungen besitzt,

welche man mit Carbonyldiimidazol und Meldrum-Säure in Gegenwart von Pyridin in Methylenchlorid behandelt zur Bildung der Verbindung der Formel V:

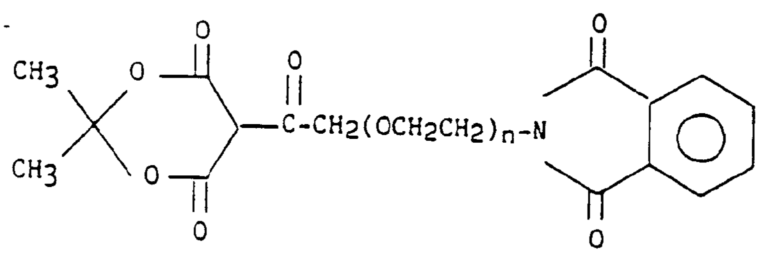

(V)

welche man anschließend mit (R)-2-Phenyl-2-methoxy-ethanol, der Verbindung der Formel VI:

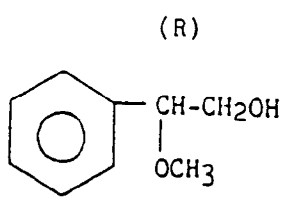

(VI)

umsetzt zur Bildung eines β-Ketoesters der Formel VII:

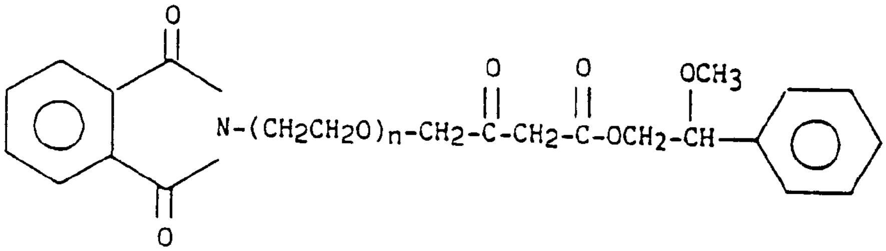

(VII)

in der n die bezüglich der Formel I angegebenen Bedeutungen besitzt,
welche man in Gegenwart von Ammoniumformiat in Ethanol mit einem Benzyliden der Formel VIII:

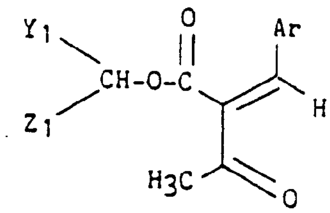

(VIII)

in der Ar, $Y_1$ und Z die bezüglich der Formel I angegebenen Bedeutungen besitzen,
kondensiert, so daß man eine Mischung von zwei Stereoisomeren einer Verbindung der Formel IX:

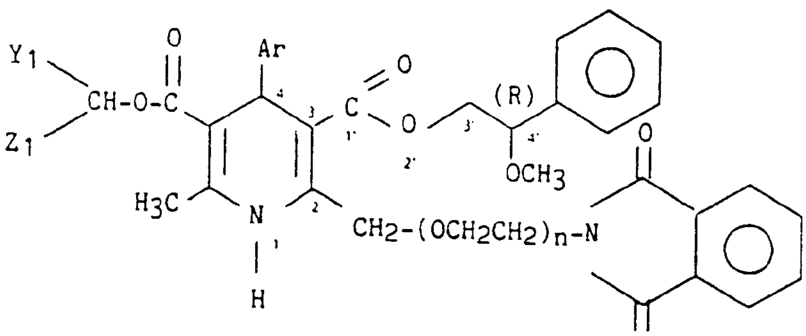

(4R,4'R/4S,4'R)

(IX)

in der Ar, $Y_1$, $Z_1$ und n die bezüglich der Formel I angegebenen Bedeutungen besitzen,
erhält, welche man anschließend:
<u>entweder</u>
der Einwirkung einer wäßrigen Lösung von Natriumbicarbonat in Lösung in Acetonitril unterwirft zur Bildung einer Mischung der Stereoisomeren einer Verbindung der Formel X:

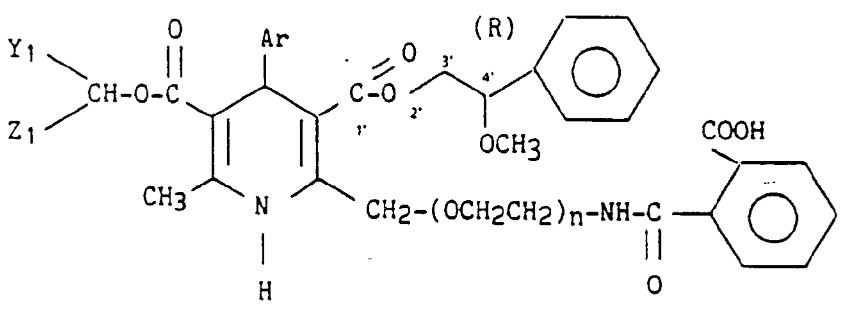

(X)

(4R,4'R/4S,4'R)

in der Ar, $Y_1$, $Z_1$ und n die bezüglich der Formel I angegebenen Bedeutungen besitzen, die man durch HPLC in die beiden Stereoisomeren auftrennt, worauf man anschließend jedes Stereoisomere getrennt mit einer Mischung Glyme und einem Alkoholat der Formel XI:

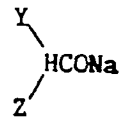

(XI)

in der Y und Z die bezüglich der Formel I angegebenen Bedeutungen besitzen, behandelt zur Bildung einer Mischung, welche eines der beiden Stereoisomeren einer Verbindung der Formel XII:

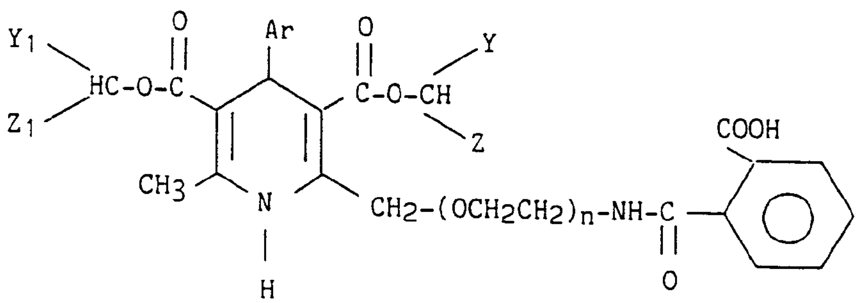

(XII)

in der Y, Z, $Y_1$, $Z_1$, Ar und n die bezüglich der Formel I angegebenen Bedeutungen besitzen,

sowie dessen in der 5-Stellung durch eine Ethoxycarbonylgruppe substituiertes Homologes, enthält,

oder

man die Verbindung der Formel IX durch Chromatographie über eine mit Siliciumdioxid beschickte Säule in die beiden Stereoisomeren auftrennt und anschließend jedes Stereoisomere getrennt der Einwirkung eines Alkoholats der Formel XI in Gegenwart von Glyme in Lösung in Ethanol unterwirft zur Bildung einer Mischung, welches ein Stereoisomeres der Verbindung der Formel XII und dessen in der 5-Stellung durch eine Ethoxycarbonylgruppe substituiertes Homologes enthält,

worauf man die nach einer der oben angegebenen Alternativen erhaltene Mischung, die ein Stereoisomeres der Formel (XII) enthält, anschliessend der Einwirkung von Carbonyldiimidazol in Lösung in einem halogenierten Alkan bei Raumtemperatur unterwirft zur Bildung einer Mischung, die ein Stereoisomeres der Verbindung der Formel XIII:

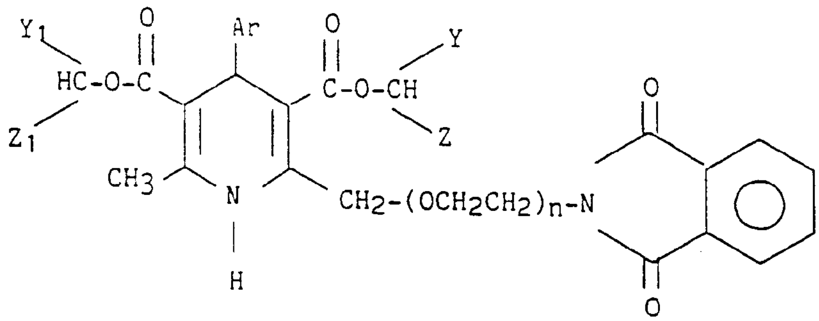

(XIII)

in der Ar, Y, Z, $Y_1$, $Z_1$ und n die bezüglich der Formel I angegebenen Bedeutungen besitzen,

und sein in der 5-Stellung durch eine Ethoxycarbonylgruppe substituiertes Homologes enthält, welche Mischung anschließend durch HPLC getrennt wird, so daß man eines der reinen Stereoisomeren der Verbindung der Formel XIII erhält,

welches anschließend in Gegenwart von Hydrazinhydrat in Ethanol zum Sieden am Rückfluß erhitzt wird zur Bildung eines der Stereoisomeren der Verbindung der Formel I,

welches anschließend mit einer organischen oder anorganischen, pharmazeutisch annehmbaren

Säure zur Bildung der entsprechenden Säureadditionssalze in ein Salz überführt wird.

2. Die reinen Stereoisomere einer Verbindung der Formel X:

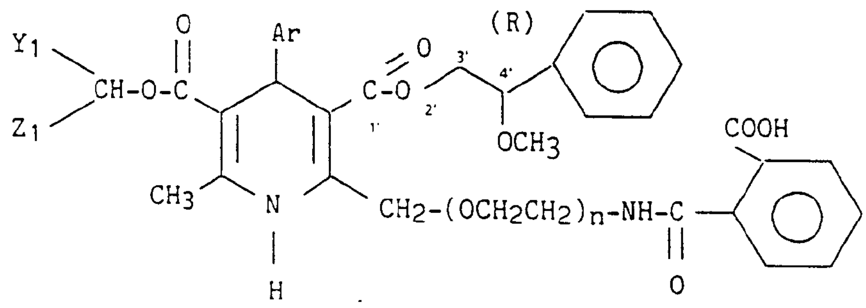

(X)

(4R,4'R/4S,4'R)

in der Ar, $Y_1$, $Z_1$ und n die in Anspruch 1 bezüglich der Formel I angegebenen Bedeutungen besitzen, als Zwischenprodukte zur Durchführung des Herstellungsverfahrens gemäß Anspruch 1.

3. Verfahren nach Anspruch 1, **dadurch gekennzeichnet**, daß man eine Verbindung der Formel XIII:

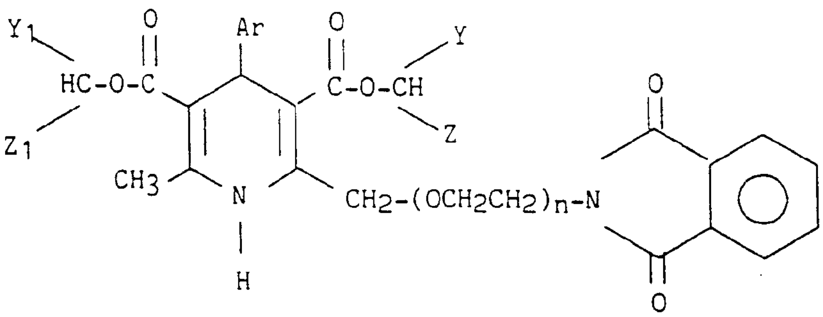

(XIII)

in der Ar, Y, Z, $Y_1$, $Z_1$ und n die bezüglich der Formel I in Anspruch 1 angegebenen Bedeutungen besitzen,

erhält durch Umsetzen einer Verbindung der Formel XII:

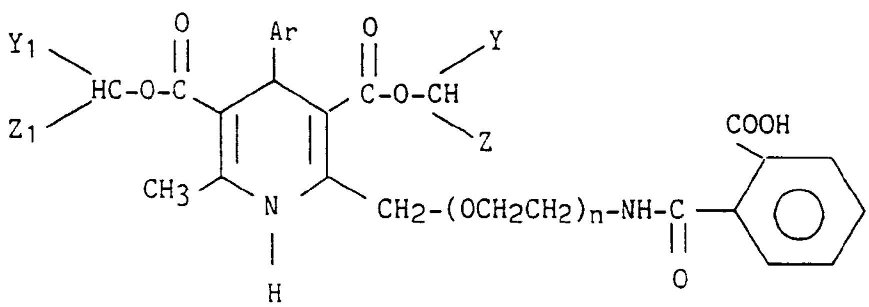

(XII)

in der Y, Z, $Y_1$, $Z_1$, Ar und n die in Anspruch 1 bezüglich der Formel I angegebenen Bedeutungen besitzen,

mit Carbonyldiimidazol in Lösung in einem halogenierten Alkan, wie Methylenchlorid bei Raumtemperatur.

## Claims

1. Process for the preparation of optical isomers of the compounds of the general formula I:

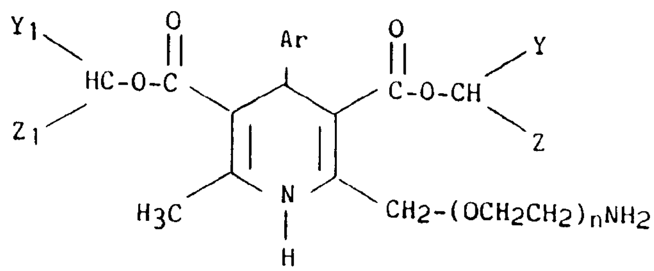

(I)

in which:

- Ar represents a phenyl radical optionally containing from one to five identical or different substituents each representing a halogen atom, an alkoxy radical containing from 1 to 4 carbon atoms, an alkylthio radical containing from 1 to 4 carbon atoms, a trihalomethyl radical, or a methylenedioxy radical,
- each of Y, Z, $Y_1$ and $Z_1$, which may be identical or different, represents a hydrogen atom, a linear or branched lower alkyl radical containing from 1 to 4 carbon atoms, a cyclopropyl radical, a dicyclopropylmethyl radical, a 2,2-dicyclopropylethyl radical, a 2,2-dicyclopropylethenyl radical, a 3,3-dicyclopropylpropyl radical, a 3,3-dicyclopropyl-1-propenyl radical, a linear or branched alkenyl radical containing from 2 to 5 carbon atoms, or a phenyl radical optionally substituted by a nitro radical, and
- n is 1, 2 or 3,

characterised in that a compound of the general formula II:

$$OH\text{-}CH_2\text{-}CH_2\text{-}(OCH_2CH_2)_n\text{-}Cl \qquad (II),$$

in which n has the same meaning as for formula I,
is condensed with potassium phthalimide, in hot dimethylformamide, to form a compound of formula III:

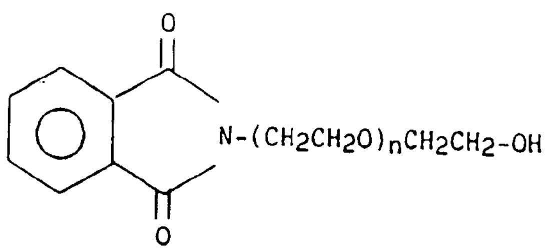

(III),

in which n has the same values as for formula I,
which is converted with the aid of the Jones' reagent into a compound of formula IV:

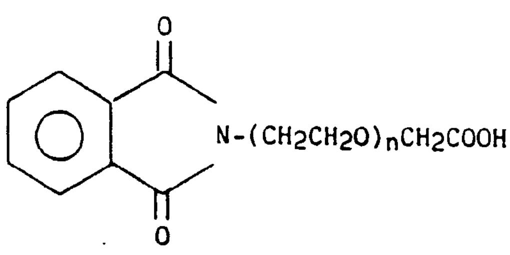

(IV),

in which n has the same meaning as for formula I,
which is treated with carbonyldiimidazole and Meldrum acid in the presence of pyridine in methylene chloride, to obtain the compound of formula V:

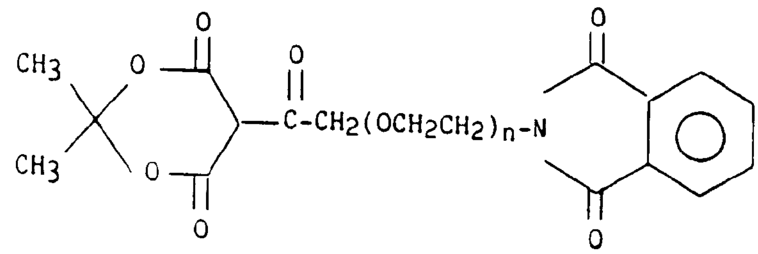

(V),

which is then reacted with 2(R)-phenyl-2-methoxyethanol, compound of formula VI:

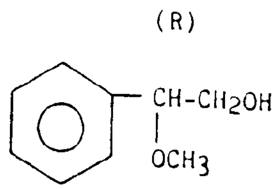

(VI),

to obtain a β-keto ester of formula VII:

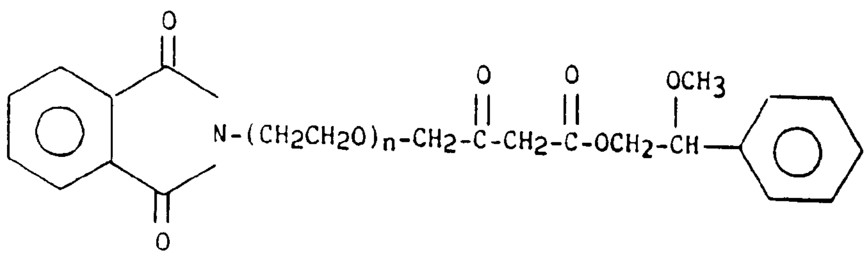

(VII),

in which n has the same meaning as for formula I,
which is condensed in the presence of ammonium formate in ethanol, with a benzylidene of formula VIII:

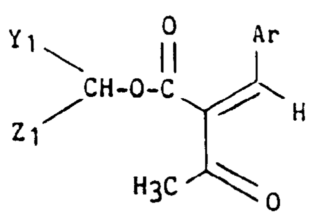

(VIII),

in which the meaning of Ar, $Y_1$ and $Z_1$ is the same as that given for formula I,
to obtain a mixture of two stereoisomers of a compound of formula IX:

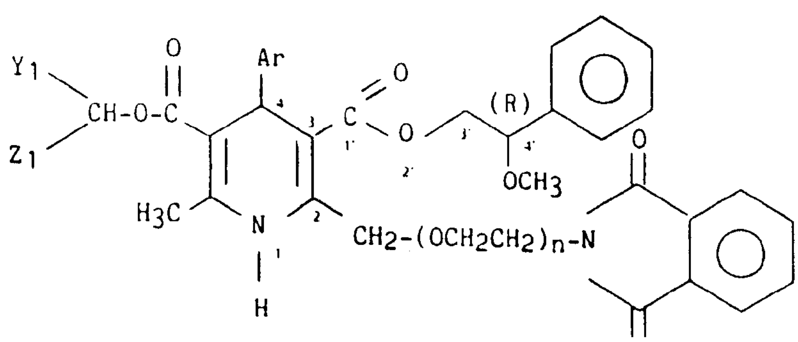

(IX),

(4R,4'R/4S,4'R)

in which Ar, $Y_1$, $Z_1$ and n have the same meanings as for formula I,
which is then:
either
subjected to the action of an aqueous sodium hydrogen carbonate solution, in solution in acetonitrile, to obtain a mixture of stereoisomers of a compound of formula X:

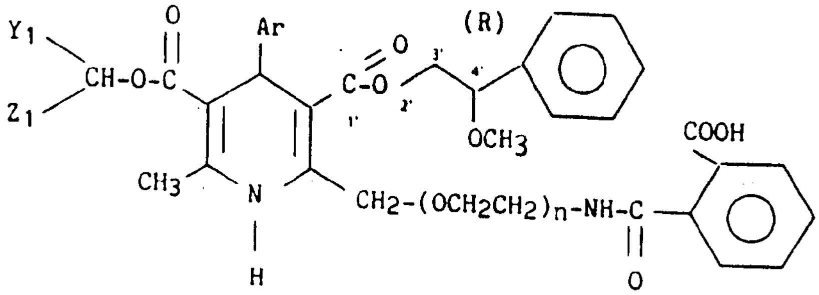

(X),

(4R,4'R/4S,4'R)

in which Ar, $Y_1$, $Z_1$ and n have the meanings given above for formula I,
which is separated by means of HPLC into its two stereo-isomers, and each stereoisomer is then treated separately with a mixture of glyme and an alcoholate of formula XI:

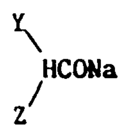

(XI),

in which the meaning of Y and Z is the same as that given for formula I,
to obtain a mixture containing one of two stereoisomers of a compound of formula XII:

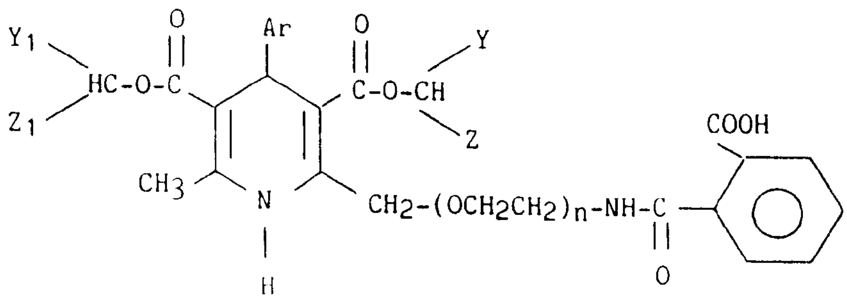

(XII),

in which Y, Z, $Y_1$, $Z_1$, Ar and n have the meanings given for formula I,
as well as its homologue substituted in the 5-position by an ethoxycarbonyl radical,
or
the compound of formula IX is separated into its two stereoisomers by chromatography over a silica column, and then each stereoisomer is subjected, separately, to the action of an alcoholate of formula XI in the presence of glyme, in solution in ethanol, to obtain a mixture containing a stereoisomer of the compound of formula XII, and its homologue substituted in the 5-position by an ethoxycarbonyl radical, then the said mixture containing a stereoisomer of formula XII obtained by one of the above alternatives is subjected to the action of carbonyldiimidazole in solution in a halogenated alkane at room temperature to obtain a mixture containing a stereoisomer of the compound of formula XIII:

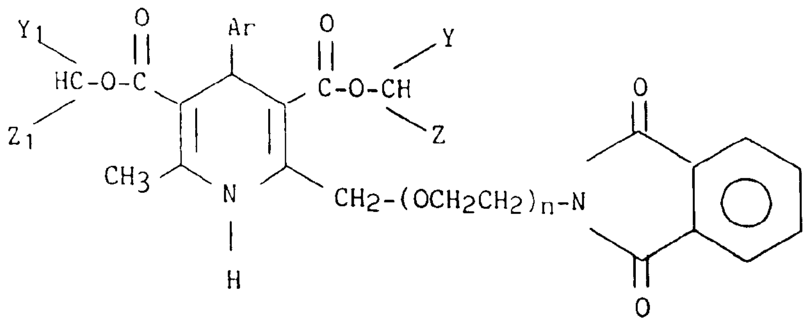

(XIII),

in which Ar, Y, Z, $Y_1$, $Z_1$ and n have the same meaning as for formula I,
and its homologue substituted in the 5-position by an ethoxycarbonyl radical, which mixture is then separated by means of HPLC to obtain one of the stereoisomers of the pure compound of formula XIII,
which is then refluxed in ethanol in the presence of hydrazine hydrate to give one of the stereoisomers of the compound of formula I,
which may then be converted into a salt with a pharmaceutically compatible mineral or organic acid to obtain the corresponding addition salts.

2. The pure stereoisomers of a compound of formula X:

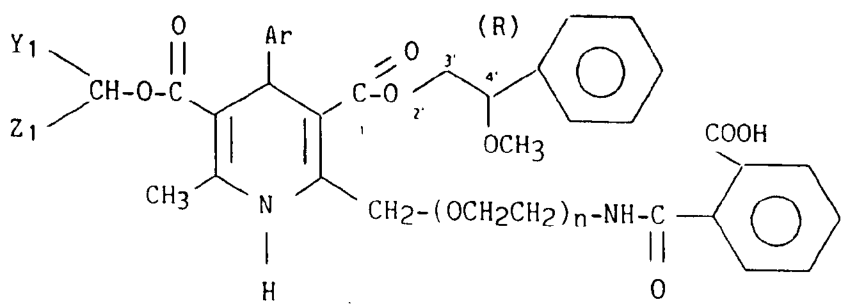

(X),

(4R,4'R/4S,4'R)

in which Ar, $Y_1$, $Z_1$ and n have the same meaning as for formula I according to claim 1, useful intermediates for the preparation process according to claim 1.

3. Process according to claim 1, characterised in that a compound of formula XIII:

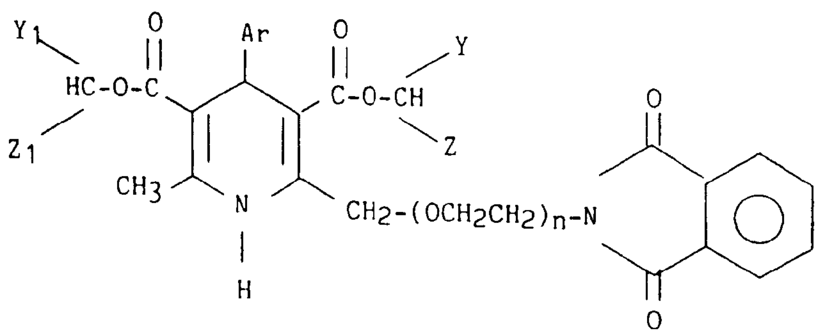

(XIII),

in which Ar, Y, Z, $Y_1$, $Z_1$ and n have the same meaning as for formula I according to claim 1,
is obtained by subjecting a compound of formula XII:

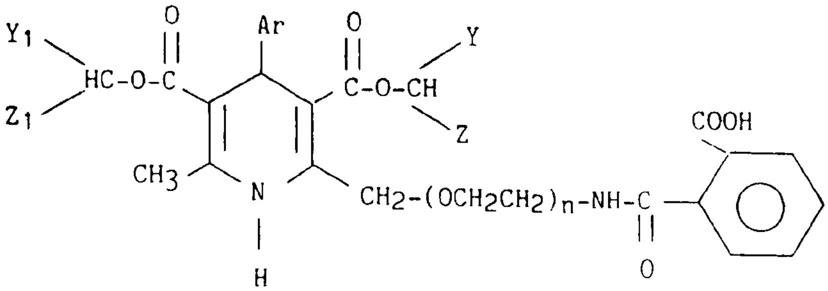

(XII),

in which Y, Z, $Y_1$, $Z_1$, Ar and n have the meanings given for formula I according to claim 1, to the action of carbonyldiimidazole in solution in a halogenated alkane, such as methylene chloride, at room temperature.